# FASCICULE DE BREVET EUROPEEN

(11) **EP 0 733 609 B1**
(45) Date de publication et mention de la délivrance du brevet: **13.11.2002**
(21) Numéro de dépôt: 96400485.7
(22) Date de dépôt: 08.03.1996
(51) Int. Cl.: C07C 11/22, C07C 4/04, C10G 9/24

(54) **Procédé de conversion thermique d'hydrocarbures aliphatiques saturés ou insaturés en hydrocarbures acétyléniques**
Verfahren zur thermischen Umsetzung von aliphatischen gesättigten oder ungesättigten Kohlenwasserstoffen in acetylenischen Kohlenwasserstoffen
Process for the thermal conversion of aliphatically saturated or unsaturated hydrocarbons in acetylenic hydrocarbons

(30) Priorité: 23.03.1995 FR 9503534
(43) Date de publication de la demande: 25.09.1996
(73) Titulaire: INSTITUT FRANCAIS DU PETROLE, 92502 Rueil-Malmaison (FR)
(72) Inventeur: Busson, Christian, F-69260 Charbonniere (FR); Alagy, Jacques, F-69260 Charbonnieres (FR); Broutin, Paul, F-69630 Chaponost (FR); Chevron, François, F-69003 Lyon (FR)
(74) Mandataire: Andreeff, François

(56) Documents cités:
- EP-A- 0 542 597
- BE-A- 612 415
- FR-A- 1 325 121

## Description

L'invention concerne un procédé de conversion thermique d'hydrocarbures aliphatiques saturés ou insaturés ayant au moins deux atomes de carbone acétylène.

Dans l'art antérieur, le procédé au carbure de calcium était la seule voie de fabrication d'acétylène jusqu'en 1940, ensuite apparurent les procédés de craquage thermique utilisant le méthane et d'autres hydrocarbures. Les premiers procédés utilisaient l'arc électrique, puis dans les années 50 furent développés les procédés régénératifs et par oxydation partielle.
Une description très complète des divers procédés est donnée dans Ullmann's Encyclopedia of Industrial Chemistry, ed. Wolfgang Gerhartz, Vol A. 1. p. 97.

Ainsi sont décrits par exemple :
- les procédés par combustion partielle tels que le procédé BASF où la chaleur est apportée par combustion d'une partie de l'hydrocarbure. L'inconvénient d'un tel procédé est la formation d'importantes quantités de CO + H_{2,}
- un procédé électrique typique est le procédé HÜLS. C'est un procédé gros consommateur d'énergie. Par ailleurs, on contrôle mal la température et le temps de séjour moyen, ce qui conduit à la formation importante de suie,
- comme procédé régénératif, citons le procédé WULLF. La chaleur est fournie par une masse de contact en matériau réfractaire à haute température qui transmet celle-ci aux hydrocarbures à craquer. Quand la température de cette masse est trop basse, elle est réchauffée par combustion de la charge ou d'un tout autre combustible. C'est un procédé difficile à conduire car la durée d'un cycle est de 1 minute.

Les gaz issus de ces procédés sont à très haute température (1 300°C environ). Pour éviter une dégradation de l'acétylène formé, ces procédés utilisent un refroidissement direct pour refroidir rapidement les gaz craqués. Il s'ensuit une perte de rendement thermique importante puisque la chaleur du gaz effluent n'est pas récupérée. La fabrication d'acétylène est également suggérée dans le brevet européen N° 0 542 597 par craquage de diverses coupes C₄. Mais ici, les charges sont exemptes d'éthane.

Dans la présente invention, il est proposé de récupérer la chaleur des gaz issus du réacteur par échange indirect, ce qui permet soit de préchauffer la charge, soit de produire de la vapeur haute pression. Ce schéma de récupération de vapeur s'apparente à celui utilisé en vapocraquage. L'utilisation d'échangeurs de chaleur crée une perte de charge qui impose donc de faire le craquage sous pression. Il est connu que la pression est néfaste pour le rendement notamment en acétylène. On a découvert que ce rendement pouvait être conservé quelle que soit la pression en utilisant comme diluant de l'eau et en ajustant le ratio eau/hydrocarbure(s) à l'entrée du réacteur en fonction de cette pression.

Les charges hydrocarbonées utilisables sont, à titre d'exemples non limitatifs des charges riches en éthane:
- les hydrocarbures aliphatiques saturés tels que l'éthane, des mélanges d'alcanes (LPG), des coupes pétrolières telles que les naphtas, les gazoles atmosphériques et les gazoles sous vide, ces derniers pouvant présenter un point final de distillation de l'ordre de 570°C,
- les hydrocarbures insaturés tels que l'éthylène, le propylène, le butadiène, des mélanges d'alcanes et d'alcènes tels que éthane + éthylène, les coupes C₃, C₄ et C₅ de vapocraquage et de craquage catalytique.
   Sur un plan industriel, les hydrocarbures préférés sont issus du vapocraquage et sont l'éthane pouvant contenir de l'éthylène en quantité très variable mais généralement assez faible, puis l'éthylène en mélange avec les autres hydrocarbures présents dans I'effluent du vapocraqueur. On peut aussi utiliser comme charge de pyrolyse, l'effluent provenant du vapocraquage d'éthane avant ou après récupération de chaleur sur ledit effluent.

Ainsi l'invention concerne un procédé d'obtention d'acétylène par pyrolyse d'au moins un hydrocarbure aliphatique saturé ou insaturé possédant au moins deux atomes de carbone par molécule, caractérisé en ce que l'on opère en présence de vapeur d'eau, sous une pression comprise entre 1,3 et 15 bar (0,1 et 1,5 MPa), le rapport pondéral eau/hydrocarbure(s) étant compris entre 2,50 et 20.

Dans une forme préférée de réalisation de la présente invention, la pression sera comprise entre 1,3 et 2 bar (0,13 à 0,2 MPa).

Généralement, pour une pression comprise entre 1,3 et 2 bar abs. 0,13 - 0,2 MPa), le ratio pondéral eau/hydrocarbure(s) est habituellement de 2,5 à 10.

Elle peut être plus précisément entre 1,3 et 1,5 bar abs (0,13 - 0,15 MPa), le ratio pondéral eau/hydrocarbure(s) étant de préférence de 2,5 à 4.

Elle peut être comprise entre 1,3 et 3 bar abs. (0,13 - 0,3 MPa), le ratio pondéral eau/hydrocarbure(s) est alors 4 à 10.

De préférence, la température finale en sortie de la zone de réaction est supérieure à 1 100°C. En général, cette température est de l'ordre de 1 200°C à 1 300°C.

Pour la mise en oeuvre du procédé selon l'invention, on peut utiliser toute technologie classique, par exemple, une technologie conventionnelle type vapocraquage sévère utilisant des matériaux métalliques résistant à plus haute température que les matériaux actuels (Incoloy), une technologie de transfert de chaleur par contact avec un solide chaud (type FCC mais à très haute température) ou par contact avec un sel fondu, etc.

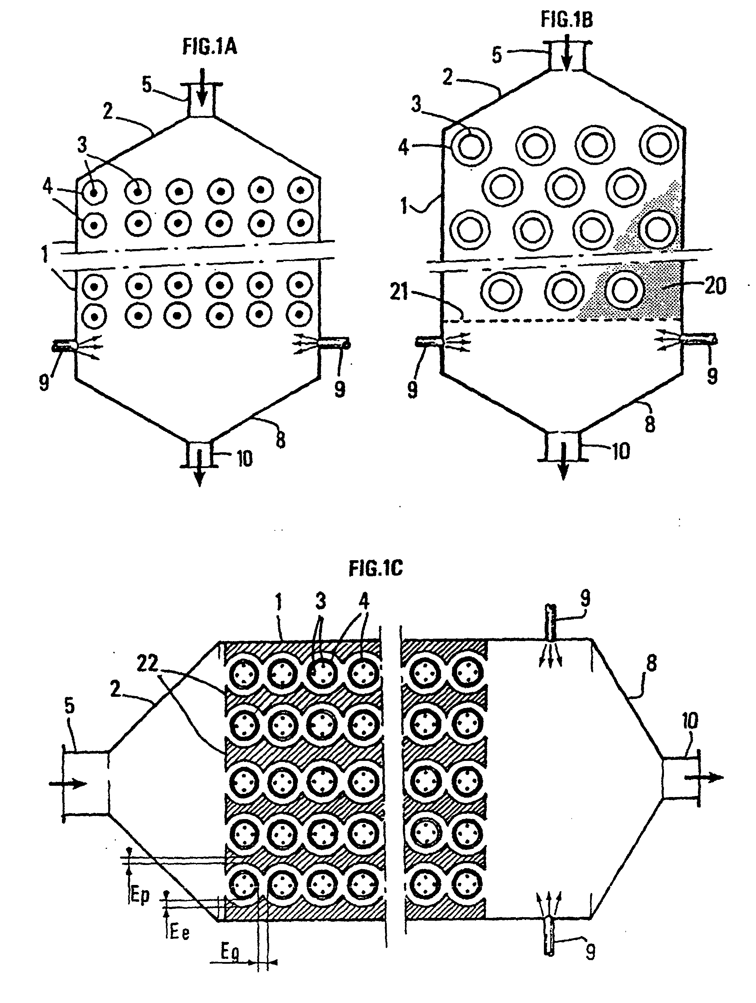

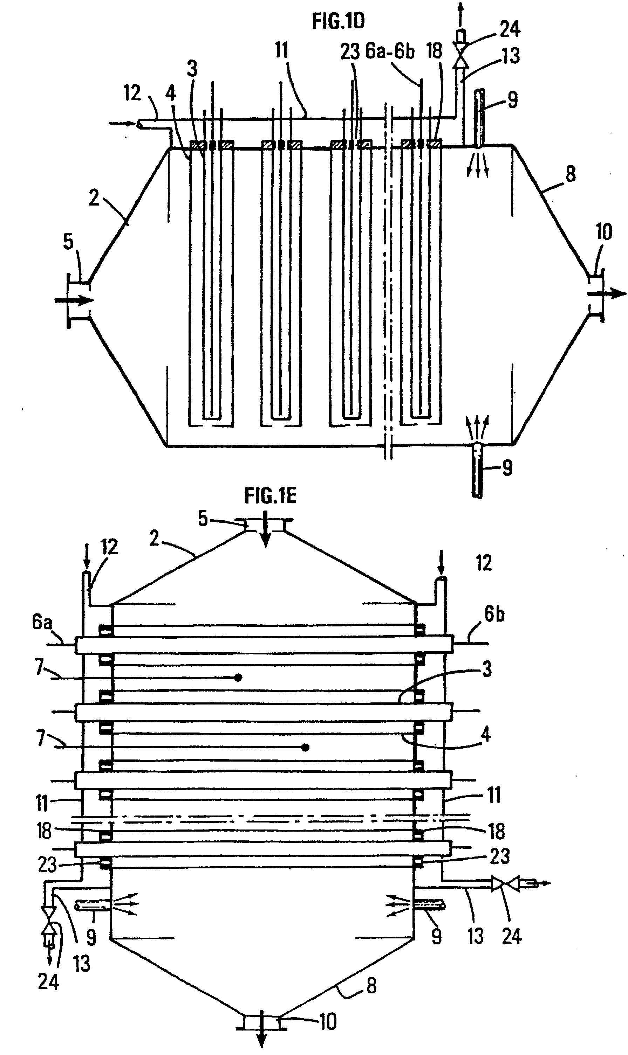

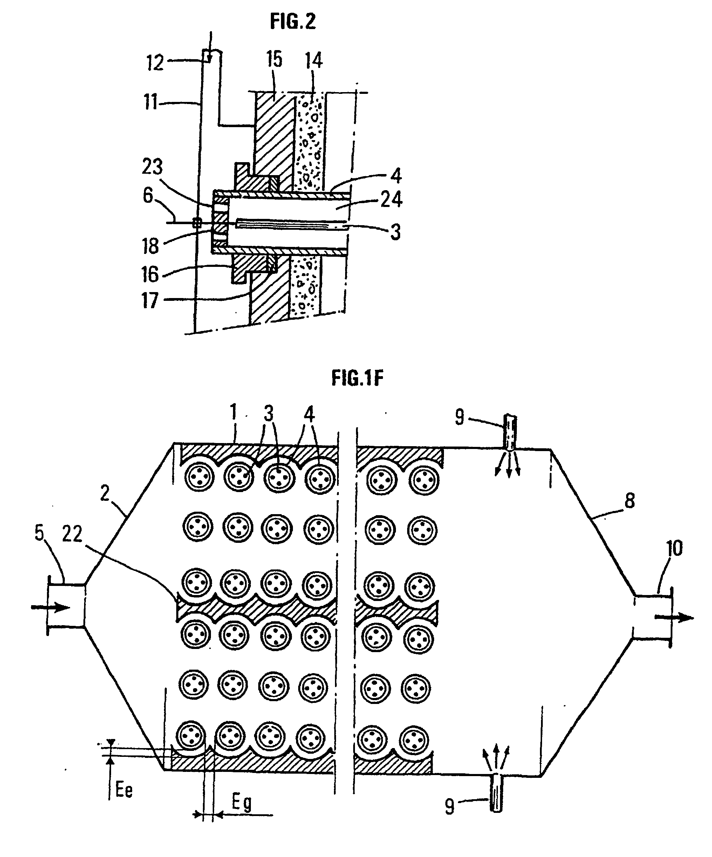

D'une façon plus particulière, à titre d'exemple non limitatif, le procédé selon l'invention peut être mis en oeuvre dans un dispositif comprenant (figure 1) un réacteur (1) de forme allongée selon un axe, de préférence à section carrée ou rectangulaire, comportant à une première extrémité des moyens d'alimentation (5) en mélange gazeux, renfermant au moins un hydrocarbure, à l'extrémité opposée des moyens d'évacuation (10) des effluents produits et entre ces deux extrémités des moyens d'alimentation en fluide de refroidissement, ledit réacteur comprenant dans une première parfle (côté première extrémité) une pluralité de moyens de chauffage électrique (3) entourés de gaines (4), lesdits moyens sensiblement parallèles entre eux étant disposés en nappes sensiblement parallèles, perpendiculaires à l'axe du réacteur de façon à définir entre les gaines et/ou les nappes formées par ces gaines des espaces ou passages pour la circulation des mélanges gazeux et/ou des effluents, lesdits moyens de chauffage et lesdites gaines étant adaptés à chauffer lesdits passages par sections transversales successives, indépendantes et sensiblement perpendiculaires à l'axe du réacteur, ledit réacteur comportant en outre des moyens d'asservissement et de modulation de chauffage reliés aux dits moyens de chauffage, et comportant dans une deuxième partie (8) (côté extrémité opposée) contiguë à la première partie des moyens de refroidissement (9) des effluents reliés aux dits moyens d'alimentation en fluide de refroidissement, ledit dispositif comprenant des moyens d'introduction, à une pression appropriée, d'un gaz G dit gaz de gaine ou gaz d'étanchéité, contenant par exemple de la vapeur d'eau et/ou de l'hydrogène et/ou de l'azote, à l'intérieur des gaines (4) et en ce que lesdites gaines ont une perméabilité suffisante pour permettre, au moins en certains points, la diffusion d'au moins une partie de ce gaz G depuis l'intérieur desdites gaines vers l'extérieur desdites gaines, ce gaz G se diluant alors dans ledit mélange gazeux. Le plus souvent on préfere utiliser l'azote comme gaz de gaine.

Le procédé de la présente invention est habituellement mis en oeuvre dans une zone de réaction, de forme allongée selon une direction (un axe), comprenant une zone de chauffage et une zone de refroidissement, faisant suite à ladite zone de chauffage, dans lequel on fait circuler, dans la zone de chauffage, un mélange gazeux, renfermant au moins un hydrocarbure à au moins deux atomes de carbone, selon une direction d'écoulement sensiblement parallèle à la direction (à l'axe) de la zone de réaction, ladite zone de chauffage comportant une pluralité de moyens de chauffage électrique disposés en nappes, sensiblement parallèles entre elles, formant en projection transversale un faisceau à pas triangulaire, carré ou rectangulaire, lesdits moyens de chauffage étant regroupés par sections successives transversales sensiblement perpendiculaires à la direction (à l'axe) de la zone de réaction, indépendantes entre elles et alimentées en énergie électrique de façon à déterminer au moins deux zones de chauffage, permettant de porter la charge jusqu'à une température au plus égale à environ 1 300°C en sortie de la dernière zone, dans lequel on refroidit les effluents de la zone de chauffage, puis on recueille les produits formés à l'extrémité de la zone réactionnelle, et dans lequel lesdits moyens de chauffage électrique sont isolés du contact direct avec le mélange gazeux renfermant au moins un hydrocarbure par des gaines dans lesquelles on introduit un gaz G dit gaz de gaine ou gaz d'étanchéité, lesdites gaines ayant une perméabilité appropriée et le gaz étant introduit à l'intérieur des dites gaines à une pression telle qu'il y a diffusion, au moins en certains points, d'au moins une partie de ce gaz G depuis l'intérieur des dites gaines vers l'extérieur des dites gaines, ce gaz G pouvant se diluer alors dans ledit mélangé gazeux.

La technologie décrite ci-dessus, effectuée dans des conditions opératoires particulières, s'applique efficacement à la production d'hydrocarbures acétyléniques et à celle de l'acétylène ou du méthylacétylène en particulier.

Les moyens d'introduction, à une pression appropriée, du gaz G sont ceux connus de l'homme du métier. Ils peuvent comprendre par ailleurs des moyens de régulation et de contrôle des prcssions régnant à l'intérieur et à l'extérieur des dites gaines.

Lesdits moyens de refroidissement sont des moyens adaptés à refroidir par contact direct ou par contact indirect les effluents quittant la zone de chauffage.

Les gaines cntourant les résistances, habituellement de façon non jointive, peuvent être disposées de façon superposée ou en quinconce et peuvent former en projection transversale un faisceau à pas triangulaire, carré ou rectangulaire.

Le nombre total de nappes comportant des moyens de chauffage et le nombre des moyens de chauffage dans chaque gaine et par nappe ne sont pas déterminants dans le procédé ; ils sont évidemment fonction de la dimension, des moyens de chauffage, des gaines qui les entourent et, lorsqu'elles existent, des parois séparant les nappes. Les éléments de chauffage peuvent être identiques entre eux ou différents, tant par leurs dimensions que par leur puissance de chauffage. À titre d'exemple, un élément chauffant pourra comprcndre à l'intérieur de la gaine de 1 à 5 résistances et le plus souvent de 1 à 3 résistances.

Le nombre d'éléments chauffant détermine la puissance électrique maximum disponible pour un volume réactionnel donné et influe également sur le temps de séjour de la charge ; il sera choisi en fonction du débit de charge admissible, compte tenu de ces paramètres.

On peut, dans le cadre de la présente invention, réaliser l'ensemble du réacteur zone de chauffage et zone de trempe soit sous forme monobloc, soit encore par juxtaposition jointive de divers éléments de forme identique, qui sont assemblés entre eux par tout moyen utilisable comme, par exemple, à l'aide de brides.

Les moyens de chauffage électrique utilisables dans le cadre de la présente invention sont de préférence des résistances chauffantes susceptibles d'être utilisées jusqu'à des températures de l'ordre de 1 500°C.

Les gaines qui entourent les résistances, de façon à éviter un contact direct entre les mélanges gazeux de la charge et les résistances, sont, de préférence, de forme tubulaire. Ces gaines en matériau réfractaire sont habituellement soit en céramique, soit en métal fritté. On peut utiliser des céramiques comme la mullite, la cordiérite, le nitrure de silicium, le carbure de silicium, la silice ou l'alumine ; le carbure de silicium est le matériau préférentiellement choisi car il présente une bonne conductivité thermique. Dans le cas où les nappes sont séparées par des parois, le matériau choisi pour réaliser ces parois peut être le même que celui utilisé pour les gaines, mais il est souvent différent, en particulier pour des questions de coût de fabrication du four.

La distance qui sépare les éléments chauffants des gaines est fonction de la section de l'élément chauffant. Pour des moyens de chauffage dont le diamètre maximal du cercle les englobant est égal à d, on utilise des gaines habituellement tubulaires ou cylindriques de diamètre D habituellement d'environ 1,1 x d à environ 8 x d, et le plus souvent d'environ 1,2 x d à environ 4 x d.

Les éléments de chauffage sont disposés en nappes parallèles sensiblement perpendiculaires au sens d'écoulement de la charge (gaz process), préférentiellement sensiblement alignés, de façon à ce que la distance qui sépare deux gaines voisines soit la plus réduite possible, tout en tenant compte des impératifs de perte de charge admissible ; la distance entre les gaines de deux nappes voisines, ou celle entre les gaines d'une nappe et la paroi la plus proche dans le cas où les nappes sont séparées par des parois, est habituellement la même que celle entre deux gaines consécutives dans une nappe donnée.

Cette distance sera habituellement telle que les passages formés entre les gaines ou entre les gaines et la paroi la plus proche, passages dans lesquels circule le mélange gazeux renfermant des hydrocarbures auront une dimension d'environ 1 à environ 100 mm, et le plus souvent d'environ 5 à environ 40 mm.
Selon un mode de réalisation de l'invention, les espaces libres ou passages définis ci-avant, destinés à la circulation du gaz process, sont au moins partiellement occupés par des garnissages, habituellement en céramique, préférentiellement conducteurs de la chaleur. On peut ainsi, pour un type de réacteur donné, diminuer le temps de séjour de la charge dans ce réacteur tout en homogénéisant l'écoulement du mélange gazeux et en répartissant mieux la chaleur dissipée. Ces garnissages peuvent avoir des formes diverses et se présenter par exemple sous forme d'anneaux (anneaux de Raschig, de Lessing ou de Pali), de selles (selles de Berl), de barreaux, de tubes cylindriques fermés.

L'invention sera mieux comprise par la description de quelques modes de réalisation, donnés à titre purement illustratif mais nullement limitatif, qui en sera faite ci-après à l'aide des figures annexées, sur lesquelles les organes similaires sont désignés par les même chiffres et lettres de référence.
- Les figures 1A, 1B, 1C et 1F représentent une coupe longitudinale d'un réacteur suivant un plan perpendiculaire à l'axe des gaines. Dans le cas de la figure 1B, ce réacteur contient un garnissage. Dans le cas des figures 1C et 1F, ce réacteur comporte des parois séparant une ou plusieurs nappes de gaines contenant les moyens de chauffage électrique.
- Les figures 1D et 1E représentent une coupe longitudinale d'un réacteur suivant l'axe des gaines.
- La figure 2 illustre un détail de réalisation de la zone de chauffage dans un plan identique à celui des figures 1D et 1E.

Sur la figure 1A, on a représenté, selon un mode de réalisation, un réacteur (1) vertical de forme allongée et de section rectangulaire, comprenant un distributeur (2) permettant d'alimenter par un orifice d'entrée (5) le réacteur en mélange gazeux réactionnel. Ce dernier, qui contient un mélange de vapeur d'eau et d'au moins un hydrocarbure, a été préchauffé dans une zone de préchauffage conventionnelle, non représentée sur la figure, de préférence par convection. Le réacteur comprend une pluralité de moyens de chauffage électrique (3) entourés de gaines (4) disposées en nappes parallèles et formant dans un plan (plan de la figure) un faisceau à pas carré. Ces nappes définissent des sections de chauffage transversales sensiblement perpendiculaires à l'axe du réacteur défini selon la direction d'écoulement de la charge.

Ces sections de chauffage sont alimentées en énergie électrique, de façon indépendante, grâce à une paire d'électrodes (6a, 6b sur les figures 1D et 1E), des sondes pyrométriques à thermocouple (7 sur la figure 1E) sont logées dans les espaces où circule la charge entre les gaines (4) et permettent de réguler automatiquement la température de chaque section de chauffage, par un dispositif classique de régulateur et de modulateur non représenté sur la figure.

La charge est introduite dans le réacteur après avoir été préchauffée à une température d'environ 500 à 800°C. Le profil thermique à l'intérieur du réacteur est réglé par modulation de la puissance électrique foumie à plusieurs sections de chauffage.

À la sortie de la zone de chauffage du réacteur, les effluents de la réaction sont refroidis dans une zone de refroidissement (8). Ils sont mis en contact avec un agent de trempe tel que de l'eau, un hydrocarbure ou un mélange d'hydrocarbures, introduit par l'intermédiaire d'injecteurs (9), de trempe, disposés à la périphérie du réacteur (1) et reliés à une source extérieure de fluide de trempe (fluide de refroidissement) non représentée. On utilise, comme agent de trempe, de préférence la charge hydrocarbonée que l'on veut convertir en hydrocarbures acétyléniques. On peut aussi utiliser, notamment dans le cas du vapocraquage d'éthane, soit de l'éthane, soit le naphta entrant comme charge dans le vapocraqueur. L'ensemble des gaz effluents est refroidi à une température d'environ 500°C et recueilli par un orifice de sortie (10) à l'extrémité de la zone réactionnelle (1).

Selon un autre mode non illustré, les effluents peuvent être refroidis en circulant à travers des conduits étanches disposés dans la zone (8) par lesquels s'écoule l'agent de trempe, ces conduits étant reliés à la source extérieure de l'agent de trempe.

Selon le mode de réalisation schématisé sur la figure 1B, le réacteur, identique à celui schématisé sur la figure 1A, comporte dans l'espace où circule la charge un garnissage (20), avantageusement en matière céramique, qui est retenu par une grille (21) à l'extrémité de la zone de chauffage. Les gaines (4) sont disposées en nappes parallèles et forment dans un plan (plan de la figure) un faisceau à pas triangulaire (disposition en quinconce).

Sur la figure 1C, on a représenté, selon un mode de réalisation, un réacteur (1) horizontal de forme allongée et de section rectangulaire, qui ne diffère du réacteur représenté sur la figure 1A que par le fait qu'il est sensiblement horizontal, qu'il comporte des gaines disposées en nappes parallèles et formant dans un plan (plan de la figure) un faisceau à pas carré, et en ce que ces nappes sont séparées les unes des autres par des parois (22) avantageusement en matière céramique. Ces parois ont une forme, adaptée à créer des turbulences, comportant des alvéoles au niveau de chaque gaine (4).

Le mode de réalisation schématisé sur la figure 1F ne différe de celui schématisé sur la figure 1C qu'en ce que plusieurs nappes d'éléments chauffants sont situées entre deux parois (22).

La figure 1D représente, pour un réacteur horizontal, les mêmes éléments que ceux décrits en liaison avec la figure 1A ; on a représenté, de plus, un boîtier de protection (11) comportant un orifice (12) par lequel on introduit le gaz G contenant par exemple de l'azote et un orifice (13) muni d'une vanne (24) permettant de réguler le flux de ce gaz G. Ce boîtier (11) est fixé sur l'armature métallique du réacteur (1) et entoure l'ensemble des résistances électriques et des gaines les contenant, à l'exception de l'extrémité des résistances électriques par où se fait l'alimentation en énergie électrique. Les résistances (3) par exemple, en épingle, sont positionnées dans les gaines (4) à l'aide de rondelles (18), par exemple en fibre céramique, comportant des passages (23) permettant au gaz G, par exemple de l'azote, de pénétrer dans l'espace compris entre les résistances et les gaines.

La figure 1E représente les mêmes éléments que ceux décrits en liaison avec la figure 1A; on a représenté, de plus, les boîtiers de protection (11) munis d'orifice (12) et (13) permettant la circulation dans les boîtiers du gaz G contenant par exemple de l'azote qui pénètre dans l'espace des résistances par les orifices (23) des rondelles (18) assurant le positionnement des résistances. Les orifices (13) sont munis de vannes (24) permettant une régulation plus facile du flux du gaz G contenant par exemple de l'azote. Ces boîtiers (11) sont fixés sur l'armature métallique du réacteur et entourent l'ensemble des résistances électriques et des gaines les contenant, à l'exception de l'extrémité des résistances électriques par où se fait l'alimentation en énergie électrique. La circulation du gaz G est effectuée en légère surpression par rapport à la pression du gaz process au sein du réacteur, assurant ainsi une atmosphère parfaitement contrôlée et une meilleure diffusion de ce gaz G vers l'espace process.

La différence de pression absolue entre l'espace des résistances et l'espace process, ou surpression, sera de préférence telle que la pression dans l'espace des résistances soit supérieure d'au moins 0,1 % et le plus souvent d'au moins 1 % à la pression dans l'espace process. Il n'est pas nécessaire d'avoir une très grande surpression et le plus souvent la pression dans l'espace des résistances reste inférieure à 2 fois la pression dans l'espace process.

La figure 2 représente un détail d'un mode de réalisation de la zone de chauffage suivant l'invention. On utilise comme moyen de chauffage électrique des résistances (3) de forme cylindrique. Ces résistances comportent à chacune de leur extrémité des zones froides et une partie de la zone centrale qui est la zone chaude représentant par exemple environ 68 % de la longueur totale.

On réalise un réacteur de section rectangulaire, dont les parois sont constituées de béton réfractaire isolant (14) et par une armature métallique (15). On perce dans deux parois latérales opposées un trou circulaire, dans lequel on fait passer une gaine (4), par exemple en céramique, de diamètre double de celui de la résistance électrique (3). La gaine (4) est positionnée au moyen d'un système presse étoupe (16) agissant dans une gorge au niveau de l'armature métallique sur une tresse en matière réfractaire (17), par exemple une tresse en matièrc céramique. Le Positionnement de la résistance (3) dans la gaine (4) est effectué au moyen de rondelles (18), par exemple en fibre céramique, comportant des orifices (23) permettant le passage du gaz G, contenant par exemple de l'azote, introduit dans le boîtier (11) par le conduit (12) dans l'espace des résistances (24).

La zone chaude de la résistance (3) est positionnée de façon à ce qu'elle ne pénètre pas dans l'orifice de passage à travers la paroi de béton isolant. Il n'est pas indispensable d'utiliser une tresse (17) au niveau du presse étoupe puisque celui-ci a, dans le cadre de l'invention, le rôle de moyen de positionnement et qu'il n'a pas pour but principal d'assurer une étanchéité aussi parfaite que possible entre l'intérieur et l'extérieur du réacteur. Ce presse étoupe peut d'ailleurs avantageusement être remplacé par un moyen plus simple de positionnement des gaines tel que par exemple des simples rondelles en matériau réfractaire.

On dispose ainsi d'un certain nombre de résistances chauffantes gainées dans des parois, par exemple en matière céramique, par rangées horizontales successives, ces rangées étant de préférence alignées de façon à ce que, sur les parois latérales du four, elles forment un faisceau à pas carré ou rectangulaire. Un boîtier (11), dont dépassent seulement les extrémités des résistances et/ou leur alimentation électrique (6), est parcouru par un courant de gaz G contenant par exemple de l'azote.

### EXEMPLES

On utilise un réacteur horizontal à trempe indirecte, dont les moyens de chauffage sont constitués par des résistances électriques en carbure de silicium du type Crusilite de chez KANTHAL. Ces résistances sont entourées de gaines en carbure de silicium fritté disposées concentriquement par rapport au centre du cercle englobant les résistances.

Ces gaines au nombre de 16 sont disposées en ligne perpendiculairement au sens de circulation de la charge (verticalement). La longueur de chaque résistance est de 100 mm et son diamètre de 10 mm. Les gaines en céramique ont une longueur de 110 mm, un diamètre extérieur de 50 mm et un diamètre intérieur de 42 mm. La distance séparant deux gaines voisines ou une gaine et la paroi du réacteur en béton réfractaire est de 5 mm.

La température du gaz le long du réacteur est régulée thermiquement par l'intermédiaire de thermocouples disposés dans les espaces où circule la charge.

La charge, préchauffée à 750°C, est chauffée de manière sensiblement linéaire jusqu'à 1 200°C en sortie de réacteur. Le temps de séjour est de l'ordre de 400 millisecondes.

Les gaz effluents sont refroidis rapidement dans un premier temps à 500°C par échange indirect, puis d'autres échangeurs de température permettent ensuite d'abaisser leur température à la température ambiante.

### Exemple 1

On utilise comme charge de l'éthane contenant 1,5 % poids d'éthylène dilué avec de l'eau dans un rapport pondéral vapeur d'eau/charge variable suivant les essais.

Les essais 1 et 2 sont des essais comparatifs. Les essais 3 et 4 sont des essais selon la présente invention.

| Numéro d'essai | 1 | 2 | 3 (comparatif) | 4 |
|---|---|---|---|---|
| Pression en bars abs | 1,1 | 1,4 | 1,1 | 1,5 |
| Ratio eau/hydeocarbure(s) en poids | 0,5 | 0,5 | 1,28 | 4,25 |
| Sélectivité en acétylène* | 31,2 | 26,7 | 41,8 | 42,2 |

| | | | | |
|---|---|---|---|---|
| *Sélectivité en acétylène = nombre de moles d'acétylène formées sur nombre de moles d'éthane converties X 100. | | | | |

Si l'on compare les essais et 2 aux essais 3 et 4 on remarque que l'utilisation d'un ratio pondéral eau/hydrocarbure(s) de 0,5 conduit à des sélectivités en acétylène faibles. Celles-ci sont d'autant plus faibles que la pression est élevée. Par contre la sélectivité est élevée pour les essais 3 et 4. Celle-ci est conservée malgré la différence de pression car le ratio eau/hydrocarbure(s) a été ajusté en fonction de ces pressions.

### Exemple 2

On utilise comme charge du naphta de densité d 20/4 = 0,715 et dont l'intervalle d'ébullition est compris entre 38 et 185°C dilué avec de l'eau dans un rapport pondéral azote/charge variable. Ce mélange est préchauffé à 600°C et chauffé de manière sensiblement linéaire jusqu'à 1 200°C dans le réacteur décrit cidevant. La pression absolue du mélange de gaz dans le réacteur est maintenue sensiblement constante et égale à 0,140 MPa. L'essai numéro 5 est un essai comparatif ayant pour but de montrer l'influence du ratio eau/hydrocarbure(s) et de la pression sur la sélectivité en acétylène. L'essai numéro 6 est effectué dans les conditions de la présente invention.

| Numéro d'essai | 5 | 6 |
|---|---|---|
| Pression en bar abs | 1,4 | 1,4 |
| Ratio eau/Hydrocarbure(s) en poids | 0,5 | 2,67 |
| Sélectivité en acétylène** | 23,6 | 35,7 |

| | | |
|---|---|---|
| ** Sélectivité en acétylène = nombre d'atome de carbone correspondant à l'acétylène formé sur nombre d'atome de carbone correspondant aux hydrocarbures de la charge craqués X 100. | | |

Pour une pression de 1,4 bar abs., on gagne 12,1 points de sélectivité en utilisant un ratio pondéral eau/hydrocarbure(s) de 2,67 au lieu de 0,5.

## Revendications

1. Procédé d'obtention d'acétylène par pyrolyse d'au moins un hydrocarbure aliphatique saturé ou insaturé possédant au moins deux atomes de carbone par molécule, **caractérisé en ce que** l'on opère en présence de vapeur d'eau, sous une pression comprise entre 1,3 et 15 bar (0,13 et 1,5 MPa) le rapport pondéral eau/hydrocarbure(s) étant compris entre 2,5 et 20.

2. Procédé selon la revendication 1 dans lequel ladite pression est comprise entre 1,3 et 2 bars (0,13 et 0,2 MPa) et ledit rapport entre 2,5 et 1 0.

3. Procédé selon la revendication 1 dans lequel ladite pression est comprise entre 1,3 et 1,5 bar (0,13 et 0,15 MPa) et ledit rapport entre 2,5 et 4.

4. Procédé selon la revendication 1 dans lequel ladite pression est comprise entre 1,3 et 3 bars (0,13 et 0,3 MPa) et ledit rapport entre 4 et 10.

5. Procédé selon la revendication 1 à 4 dans lequel la température finale en sortie de la zone de réaction est supérieure à 1 100°C.

6. Procédé selon l'une des revendications 1 à 4 dans lequel la température finale en sortie de la zone de réaction est supérieure à 1 150°C.

7. Procédé selon l'une des revendications de 1 à 6 **caractérisé en ce qu'**il est mis en oeuvre dans au moins une zone de réaction, de forme allongée selon une direction (un axe), comprenant une zone de chauffage et une zone de refroidissement, faisant suite à ladite zone de chauffage, dans lequel on fait circuler, dans la zone de chauffage, un mélange gazeux, renfermant au moins de l'éthane selon une direction d'écoulement sensiblement parallèle à la direction (à l'axe) de la zone de réaction, ladite zone de chauffage comportant une pluralité de moyens de chauffage électrique disposés en nappes, sensiblement parallèles entre elles, formant en projection transversale un faisceau à pas triangulaire, carré ou rectangulaire, lesdits moyens de chauffage étant regroupés par sections successives transversales sensiblement perpendiculaires à la direction (à l'axe) de 10 la zone de réaction, indépendantes entre elles et alimentées en énergie électrique de façon à déterminer au moins deux zones de chauffage, permettant de porter la charge jusqu'à une température au plus égale à environ 1 300°C en sortie de la dernière zone, dans lequel on refroidit les effluents de la zone de chauffage, puis on recueille les produits formés à l'extrémité de la zone réactionnelle et dans lequel lesdits moyens de chauffage électrique sont isolés du contact direct avec le mélange gazeux renfermant au moins un hydrocarbure par des gaines dans lesquelles on introduit un gaz G dit gaz de gaine ou gaz d'étanchéité, lesdites gaines ayant une perméabilité appropriée et le gaz étant introduit à l'intérieur des dites gaines à une pression telle qu'il y a diffusion, au moins en certains points, d'au moins une partie de ce gaz G depuis l'intérieur des dites gaines vers l'extérieur des dites gaines, ce gaz G pouvant se diluer alors dans ledit mélange gazeux.

## Claims

1. A process for producing acetylene by pyrolysis of at least one saturated or unsaturated aliphatic hydrocarbon containing at least two carbon atoms per molecule, **characterized in that** it is carried out in the presence of steam, at a pressure in the range 1.3 bar to 15 bar (0.13 MPa to 1.5 MPa), the steam/hydrocarbon ratio being in the range 2.5 to 20.

2. A process according to claim 1, in which said pressure is in the range 1.3 bar to 2 bar (0.13 MPa to 0.2 MPa) and said ratio is in the range 2.5 to 10.

3. A process according to claim 1, in which said pressure is in the range 1.3 bar to 1.5 bar (0.13 MPa to 0.15 MPa) and said ratio is in the range 2.5 to 4.

4. A process according to claim 1, in which said pressure is in the range 1.3 bar to 3 bar (0.13 MPa to 0.3 MPa) and said ratio is in the range 4 to 10.

5. A process according to any one of claims 1 to 4, in which the final temperature at the outlet from the reaction zone is more than 1100 C.

6. A process according to any one of claims 1 to 5, in which the final temperature at the outlet from the reaction zone is more than 1150 C.

7. A process according to any one of claims 1 to 6, **characterized in that** it is carried out in at least one reaction zone which is elongate in one direction (one axis), comprising a heating zone and a cooling zone, following said heating zone, in which a gaseous mixture comprising at least one hydrocarbon containing at least two carbon atoms is circulated in the heating zone in a flow direction which is substantially parallel to the (axial) direction of the reaction zone, said heating zone comprising a plurality of electrical heating means disposed in layers which are substantially parallel to each other, forming an array with a triangular, square or rectangular pattern in the transverse projection, said heating means being grouped in successive transverse cross sections which are substantially perpendicular to the (axial) direction of the reaction zone, independent of each other and supplied with electrical energy so as to determine at least two heating zones to heat the feed to a temperature of at most about 1300 C at the outlet from the last zone, in which the effluents from the heating zone are cooled then the products formed are recovered at the extremity of the reaction zone, and in which said electrical heating means are isolated from direct contact with the gaseous mixture comprising at least one hydrocarbon by sleeves into which a gas G, termed the sleeve gas or sealing gas, is introduced, said sleeves having an appropriate permeability and the gas being introduced to the interior of said sleeves at a pressure such that there is diffusion, at least at certain points, of at least a portion of gas G from the interior of said sleeves to the exterior of said sleeves, gas G thus dissolving in said gaseous mixture.

## Patentansprüche

1. Verfahren zum Erhalt von Acetylen durch Pyrolyse wenigstens eines gesättigten oder ungesättigten aliphatischen Kohlenwasserstoffs mit wenigstens zwei kohlenstoffatomen pro Molekül, **dadurch gekennzeichnet, dass** man in Gegenwart von Wasserdampf unter einem Druck zwischen1,3 und 15 bar (0,13 und 1,5 MPa) arbeitet, wobei das Gewichtsverhältnis Wasser/Kohlenwasserstoff(e) zwischen 2,5 und 20 liegt.

2. Verfahren nach Anspruch 1, bei dem der Druck zwischen 1,3 und 2 bar (0,13 und 0,2 MPa) und das Verhältnis zwischen 2,5 und 10 liegt.

3. Verfahren nach Anspruch 1, bei dem der Druck zwischen 1,3 und 1,5 bar (0,13 und 0,15 MPa) und das Verhältnis zwischen 2,5 und 4 liegt.

4. Verfahren nach Anspruch 1, bei dem der Druck zwischen 1,3 und 3 bar (0,13 und 0,3 MPa) und das Verhältnis zwischen 4 und 10 liegt.

5. Verfahren nach Anspruch 1 bis 4, bei dem die Endtemperatur am Ausgang der Reaktionszone über 1100°C liegt.

6. Verfahren nach einem der Ansprüche 1 bis 4, bei dem die Endtemperatur am Ausgang der Reaktionszone über 1150°C liegt.

7. Verfahren nach einem der Ansprüche 1 bis 6, **dadurch gekennzeichnet, dass** es in wenigstens einer Reaktionszone von länglicher Form gemäß einer Richtung (einer Achse) durchgeführt wird, die eine Heizzone und eine der Heizzone folgende Kühlzone umfasst, in dem man in der Heizzone ein Gasgemisch, welches wenigstens Ethan einschließt gemäß einer im wesentlichen zur Richtung (Achse) der Reaktionszone parallelen Umlaufrichtung zirkulieren lässt, wobei diese Heizzone über eine Vielzahl elektrischer, flächig, im wesentlichen parallel zueinander angeordneter Heizmittel umfasst, die in transversaler Projektion ein Bündel mit dreieckiger, quadratischer oder rechteckiger Teilung bilden, wobei diese Heizmittel durch aufeinanderfolgende transversale, im wesentlichen zur Richtung (Achse) 10 der Reaktionszone senkrechte Abschnitte, unabhängig voneinander zusammengelegt und mit elektrischer Energie versorgt sind, um wenigstens zwei Heizzonen zu bestimmen, die es ermöglichen, die Charge bis auf eine Temperatur oberhalb von etwa 1300°C am Ausgang der zweiten Zone zu bringen, in dem man die Abströme der Heizzone kühlt und man dann die am Ende der Reaktionszone gebildeten Produkte sammelt, und in dem die elektrischen Heizmittel vom direkten Kontakt mit dem wenigstens einen Kohlenwasserstoff einschließenden Gasgemisch durch Hüllen getrennt sind, in die man ein Hüllengas oder Isoliergas genanntes Gas G einführt, wobei die Hüllen eine geeignete Permeabilität haben und das Gas in die Hüllen bei einem Druck derart eingeführt wird, dass es wenigstens an bestimmten Punkten eine Diffusion wenigstens eines Teils dieses Gases G vom Inneren der Hüllengase aus zum Äußeren der Hüllen gibt, wobei dieses Gas sich so in dem Gasgemisch verdünnen kann.
